# EUROPEAN PATENT APPLICATION

(11) **EP 4 512 353 A1**
(43) Date of publication of application: **26.02.2025**
(21) Application number: 24195855.2
(22) Date of filing: 22.08.2024
(51) Int. Cl.: A61B 18/02, A61B 17/00, A61F 7/12

(54) **CATHETER INCLUDING INTRA-EXTRAVASULAR COOLING NEEDLES FOR CRYOGENIC THERAPY**

(30) Priority: 25.08.2023 US 202363578749 P; 14.08.2024 US 202418804389
(71) Applicant: Medtronic Ireland Manufacturing Unlimited Company, Dublin 2, D02 T380 (IE)
(72) Inventor: PANDA, Binit, Minneapolis, 55432 (US); LIMA, Carlos H., Santa Rosa, 95403 (US); PETERSON, Darion R., Boulder, 80301 (US)
(74) Representative: Zimmermann & Partner Patentanwälte mbB

(57) **Abstract**

A catheter assembly includes an elongate catheter body defining a longitudinal axis, and a plurality of cooling needles configured to be extended from the catheter body to penetrate a vessel wall of a vessel in which the elongate catheter body is positioned and extend to an ablation region within tissue adjacent the vessel wall. At least one cooling needle of the plurality of cooling needles is further configured to receive a flow of cryogenic fluid to cool the at least one cooling needle.

## Description

### RELATED APPLICATIONS

This application claims the benefit of U.S. Provisional Application Serial Number 63/578,749, filed August 25, 2023, the entire contents of each of which are incorporated herein by reference.

### FIELD

The present technology is generally related to cryogenic devices for cryogenic therapies.

### BACKGROUND

Renal denervation and similar cryotherapies are commonly performed by inflating a balloon within a vessel (e.g., the renal artery) with a refrigerant to remove heat from surrounding tissues, thereby ablating nearby material (e.g., renal nerves in or around the vessel wall of the renal artery). The balloon is supported by a catheter which provides the refrigerant to the balloon to perform the therapy. Introduction of the refrigerant to the balloon causes the balloon to expand, and the temperature within the balloon is commonly monitored throughout the therapy process.

### SUMMARY

The techniques of this disclosure generally relate to the use of devices, other than balloons, for cryotherapies, including renal denervation. Such devices include, for example, one or more intravascular cooling needles that are delivered through a catheter body to a location within the vessel. At least a distal portion of the needle penetrates the vessel wall and extends into tissue surrounding the vessel. Refrigerant is delivered through portions of the cooling needles to provide the cryogenic ablation energy for the cryotherapy, such as ablating renal nerves around a renal artery.

In one aspect, the disclosure provides a catheter assembly that includes an elongate catheter body defining a longitudinal axis, and a plurality of cooling needles configured to be extended from the catheter body to penetrate a vessel wall of a vessel in which the elongate catheter body is positioned and extend to an ablation region within tissue adjacent the vessel wall. At least one cooling needle of the plurality of cooling needles is further configured to receive a flow of cryogenic fluid to cool the at least one cooling needle.

Further disclosed herein is a catheter assembly that includes an elongate catheter body defining a longitudinal axis, and a plurality of cooling needles configured to be extended from the catheter body to penetrate a vessel wall of a vessel in which the elongate catheter body is positioned and extend to an ablation region within tissue adjacent the vessel wall, wherein at least one cooling needle of the plurality of cooling needles is further configured to receive a flow of cryogenic fluid to cool the at least one cooling needle.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 is a schematic view of an assembled catheter assembly according to one example, showing the catheter assembly positioned within a renal artery during use.
FIG. 2 is a further schematic view of the assembled catheter assembly.
FIG. 3 is a schematic view of the assembled catheter assembly, illustrating three cooling needles positioned in a stacked arrangement.
FIG. 4 is a schematic view of the assembled catheter assembly, illustrating the three cooling needles being deployed from the stacked arrangement.
FIG. 5 is a partial view of one end of one of the cooling needles, and a schematic illustration of the renal artery, illustrating different regions of the cooling needle.
FIG. 6 is a schematic, cross-sectional view of the cooling needle, taken along lines 6-6 in FIG. 5.
FIG. 7 is a schematic view of the cooling needles of a catheter assembly according to another example.

### DETAILED DESCRIPTION

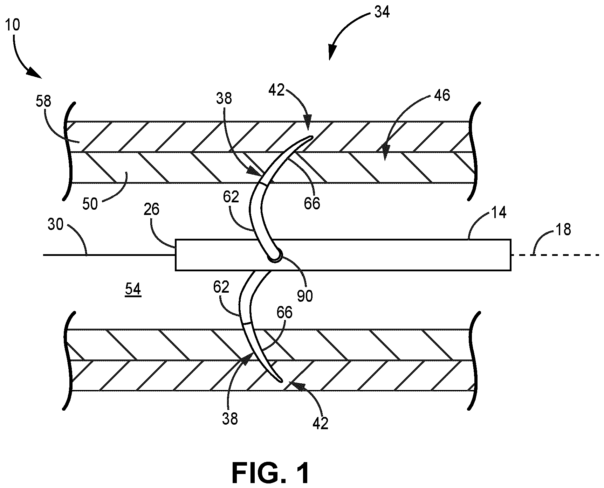

FIGS. 1-7 illustrate an example of a catheter assembly 10. The catheter assembly 10 may be used, for example, to perform renal denervation, or to perform other cryogenic ablation procedures, without the use of a balloon.

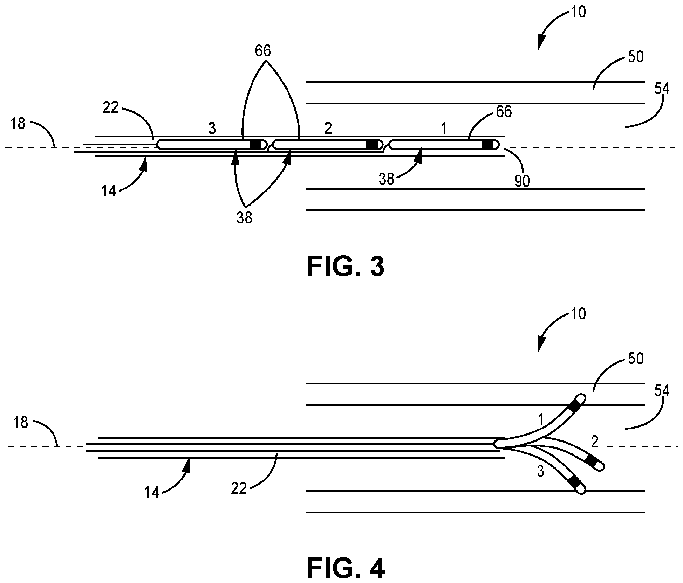

With reference to FIGS. 1-4, in the illustrated example the catheter assembly 10 includes an elongate catheter body 14 defining an axis 18 (e.g., a central, longitudinal axis) extending through the catheter body 14. The catheter body 14 defines at least one hollow interior passage 22 (illustrated schematically in FIGS. 3 and 4) for insertion and/or removal of at least one medical device(s) and/or cryogenic fluid(s) through the catheter body 14. In some examples, the catheter body 14 is flexible, and is formed at least partially of a polymer or polymers, such as polyether block amide, polyimide, and/or other suitable materials.

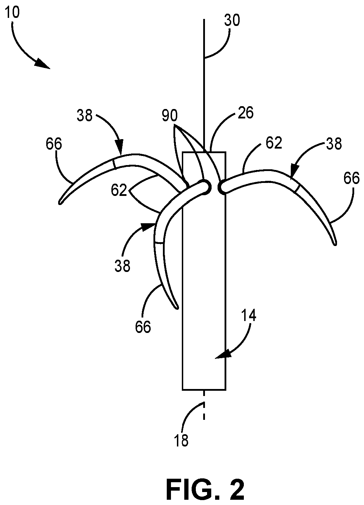

With reference to FIGS. 1 and 2, the catheter body 14 includes a distal end 26. In some examples, the catheter assembly 10 includes a guidewire 30 that is sized and shaped to extend from the distal end 26 along the axis 18. The catheter body 14 may be guided over the guidewire 30, to facilitate insertion of the catheter body 14 into a vasculature system 34 of a patient. In some examples, the guidewire 30 is formed at least partially of a metal or metal alloy (e.g., Nitinol), or other suitable material. Other catheter assemblies 10 do not use a guidewire 30.

With reference to FIGS. 1-5, the catheter assembly 10 also includes a plurality of elongate, flexible intravascular cooling needles 38, each sized and shaped to be delivered axially through the hollow interior passage 22 of the catheter body 14 to an ablation region 42 within the vasculature system 34, and to provide ablation energy to the ablation region 42. In some examples, and with reference to FIG. 1, the vasculature system 34 includes a renal artery 46 having a wall 50 (e.g., vessel wall) that defines a hollow interior 54 within the renal artery 46. The ablation region 42 is an area near and/or on the renal artery 46 having renal nerves that have become embedded within, engaged with, and/or located in proximity to the wall 50. In the illustrated construction, the renal nerves are located in an area of tissue 58 outside of the wall 50. The cooling needles 38 deliver ablation energy to the ablation region 42, to ablate the renal nerves.

With reference to FIGS. 1-7, in the illustrated example each of the cooling needles 38 includes a first section 62, and a second section 66 (e.g., defining a sharp tip) extending distally from the first section 62. The first section 62 of each cooling needle 38 is at least partially hollow, to facilitate movement of cryogenic fluid within the first section 62, whereas the second section 66 of each cooling needle 38 is solid.

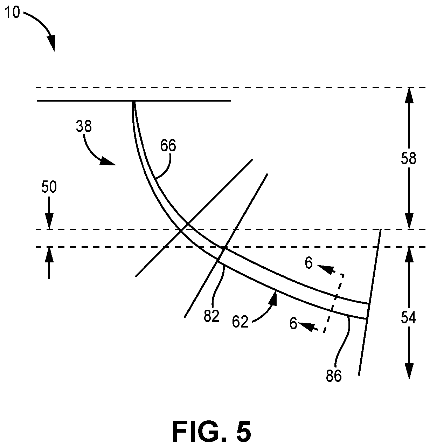

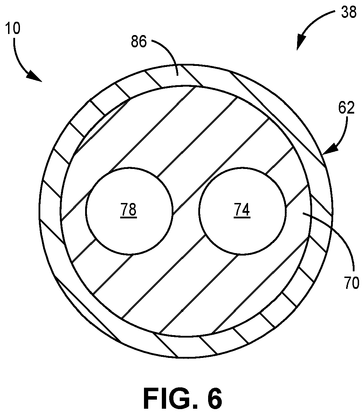

With reference to FIGS. 5 and 6, in the illustrated example, the first section 62 of the cooling needle 38 includes a body 70 (e.g., formed from polyimide or other suitable material) defining an inlet channel 74 to direct a flow of cryogenic fluid (e.g., carbon dioxide, liquid nitrogen, argon, nitrous oxide, or other refrigerants) distally through the first section 62 of the cooling needle 38 toward the second section 66. The body 70 also defines an outlet channel 78 for the cryogenic fluid to flow proximally back through the first section 62. During use, the cryogenic fluid is directed from a fluid source (not shown) through the inlet channel 74 (e.g., under a desired pressure and/or temperature) to a distal end 82 (FIG. 5) of the first section 62. The cryogenic fluid then returns back proximally through the outlet channel 78 (e.g., with the assistance of a vacuum source). In the illustrated example, the inlet channel 74 and the outlet channel 78 extend parallel to one another along at least a portion of the cooling needle 38. In other examples, the inlet channel 74 and the outlet channel 78 are arranged coaxially within the body 70, and/or are formed by separate tubes within the body 70. Other examples include other numbers, sizes, and arrangements of inlet and outlet channels 74, 78 than that illustrated.

With continued reference to FIGS. 5 and 6, in the illustrated example the inlet channel 74 and the outlet channel 78 have identical cross-sectional diameters, although in other examples the inlet channel 74 and the outlet channel 78 have different diameters from one another. Additionally, in some examples at least one of the inlet channel 74 and the outlet channel 78 has a varying cross-sectional diameter. For example, the inlet channel 74 may enlarge in cross-section as the inlet channel 74 approaches the distal end 82 of the first section 62. The inlet channel 74 may enlarge into a an open cavity, or chamber, at the distal end 82, before transitioning into the outlet channel 78. In some examples, during use of the cooling needle 38, the cryogenic fluid is intended to move distally through the inlet channel 74, and then return back proximally though the outlet channel 78, creating a continuous flow of cryogenic fluid to the distal end 82 of the first section. Depending on the type of material selected, and/or the geometry selected for the inlet and outlet channels 74, 78, the cryogenic material may or may not undergo a phase change. For example, the cryogenic fluid may undergo a phase change in regions of lower pressure (e.g., where the inlet channel 74 has increased in diameter). Where liquid nitrogen is used as the cryogenic material, the liquid nitrogen may remain liquid throughout movement within the first section 62. Nucleation may occur at the distal end 82.

With continued reference to FIGS. 5 and 6, in some examples the first section 62 includes an outer layer 86 (e.g., extending around the body 70) that is exposed within the hollow interior 54 of the renal artery 46. In the illustrated example, the outer layer 86 is a highly insulative layer (e.g., film or tape), formed for example from polyimide, or other suitable material. The outer layer 86 provides insulation against blood that is flowing within the hollow interior 54 of the renal artery 46.

With continued reference to FIGS. 5 and 6, and as described above, the second section 66 of each cooling needle 38 defines a tip of the cooling needle 38. The second sections 66 are sized and shaped to penetrate the wall 50 of the vasculature (e.g., the vessel wall of the renal artery 46). In some examples, the second sections 66 are multi-faceted (e.g., three-faceted), or have other sharpened profiles and geometries. In the illustrated example, the second sections 66 are solid, and are each formed partially or entirely from a highly conductive (e.g., thermally superconductive) material (e.g., gold) such that the second sections 66 transfer all or substantially all of the cooling taking place in the first sections 62 to the second sections 66 and to the wall 50, to cause an ablation of targeted material (e.g., the renal nerves located in the area of tissue 58).

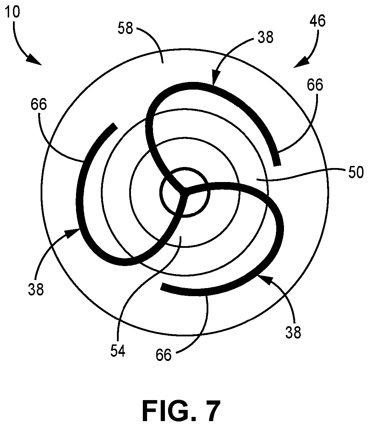

In some examples, each of the second sections 66 is sized and shaped to be deployed outwardly (e.g., through a distal end of the catheter body 14), and is configured to extend radially relative to the axis 18 and radially penetrate the wall 50. Each of the second sections 66 may also, or alternatively, be sized and shaped to extend circumferentially relative to the axis 18 and circumferentially penetrate the wall 50. For example, and with reference to FIG. 7, in some examples the cooling needles 38 include second sections 66 that are shaped to extend circumferentially within the wall 50, for example to provide cooling to a larger area within the wall 50. Deployment of the distal, second sections 66 of the cooling needles 38 out of the catheter body 14 may occur manually (e.g., via a handle or other mechanical structure at a proximal end of the catheter), and/or automatically (e.g., via a controller or other automated system associated with the cooling needles 38).

With reference to FIGS. 3 and 4, in some examples the cooling needles 38 are stacked within the catheter body 14, and/or are arranged telescopically, such that the cooling needles 38 are deployed sequentially. FIG. 3, for example, illustrates an arrangement in which a portion of a first cooling needle 38 (referenced as "1") is stacked over a portion of a second cooling needle 38 (referenced as "2"), and a portion of the second cooling needle 38 is stacked over a third cooling needle 38 (referenced as "3"). Distal ends (e.g., the second sections 66) of the three cooling needles 38 are generally aligned along a common axis when stacked. FIG. 4 illustrates deployment, during which time the first cooling needle 38 is deployed first, followed by the second cooling needle 38, followed by the third cooling needle 38. Such an arrangement may permit larger size cooling needles 38 to be used within the catheter body 14, without having to increase the size of the catheter body 14. In yet other examples, the cooling needles 38 are not stacked, but rather are arranged in a different manner (e.g., such that the distal ends remain parallel to one another, and/or symmetrically around the guidewire 30, prior to deployment).

In the illustrated example, the catheter assembly 10 includes three cooling needles 38 (only two being visible in FIG. 1). Other examples, however, include different numbers and arrangements of cooling needles 38. For example, some catheter assemblies 10 include two cooling needles 38, or four cooling needles 38, or five cooling needles 38, that together extend through the catheter body 14 and are deployed. The cooling needles 38 (or at least the second sections 66 thereof) may be arranged symmetrically (e.g., arrangement symmetrically about the guidewire 30), or may otherwise be arranged. In some examples, when the second sections 66 are deployed and exit the catheter body 14, the second sections 66 penetrate the wall 50 at locations that are equidistant from one another around a circumference of the wall 50.

With reference to FIGS. 1-7, in some examples, each of the cooling needles 38 is pre-stressed, such that the cooling needle 38 is configured to be pushed axially through the elongate catheter body 14, and the second section 66 is configured to automatically move radially and/or circumferentially relative to the axis 18 upon outward deployment from the catheter body 14. The pre-stress may take place during a manufacturing step of forming the cooling needle 38. For example, at least a portion of the first section 62 and the second section 66 of each cooling needle 38 may be pressed or otherwise mechanically shaped, to form a pre-stress within the cooling needle 38 that automatically results in a desired movement once the cooling needle 38 is deployed. Additionally or alternatively, the cooling needle 38 may be formed initially with a curved and/or enlarged profile. Forcing the cooling needle 38 into the shape of the hollow interior passage 22 of the catheter body 14 may result in the desired pre-stress, such that when the cooling needle 38 is moved axially and deployed, the first section 62 and/or second section 66 may automatically attempt to reassume its curved or enlarged state, thus causing the sharped tips defined by the second sections 66 to move radially and/or circumferentially (e.g., at various angles relative to the axis 18) and penetrate the wall 50 of the vasculature system 34. For example, each of the cooling needles 38 (e.g., each of three cooling needles 38 if three cooling needles 38 are provided) may be pre-stressed in a coiled configuration, such that the cooling needle 38 is configured to be pushed axially through the elongate catheter body 14 and the second section 66 is configured to automatically move both radially and circumferentially relative to the axis 18 upon outward deployment from the catheter body 14.

With reference to FIGS. 1 and 2, and as described above, the cooling needles 38 may be deployed out of the catheter body 14 (e.g., at a distal end of the catheter body). In some examples, therefore, the distal end of the catheter body 14 defines separate apertures 90 for each cooling needle 38 (e.g., three separate apertures 90). The second section 66 of each cooling needle 38 is sized and shaped to be deployed out of the catheter body 14 through a different one of the three separate apertures 90. In yet other examples, the distal end of the catheter body 14 defines just a single aperture 90, and the plurality of cooling needles 38 (e.g., three cooling needles 38) include second sections 66 that are sized and shaped to be deployed out of the catheter body 14 through the single aperture 90.

With reference to FIGS. 1 and 5, during one example of use, the catheter body 14 is first guided over the guidewire 30 to the ablation region 42. At least a portion of the material of the catheter assembly 10 (e.g., near the distal end 26) may be made a radiopaque material, such that X-ray imaging (e.g., an angiogram) may be used to track a location of the distal end 26 of the catheter body 14. Additionally or alternatively, in some examples an ultrasound and/or marker bands may be used to track a location of the catheter body 14. Once the catheter body 14 is tracked and positioned at a desired location next to the ablation region 42, the cooling needles 38 are deployed. During the deployment, the second sections 66 of the cooling needles 38 extend out radially and/or circumferentially within the hollow interior 54 of the renal artery 46, until the second sections 66 pierce and penetrate the wall 50. The second sections 66 continue to extend radially and/or circumferentially into the wall 50 and/or into the area of tissue 58, and to locations at or near the renal veins. In some examples, the second sections 66 penetrate at least a depth of 1 mm radially into the wall 50, or between 1 mm - 3 mm radially into the wall 50, or between 0.5 mm - 6 mm radially into the wall 50. In some examples, the second sections 66 pierce past the vessel wall 50, and into the area of tissue 58 outside of the wall 50. For example, and as illustrated in FIG. 5, in some examples a majority of a length of the second section 66 extends into the area of tissue 58. Due to the flexibility and movement of the cooling needles 38, the cooling needles 38 may be used in arteries and other vessels of various sizes and dimensions, to penetrate a wall 50 of the vessel and target specific areas for ablation.

With continued reference to FIGS. 1 and 5, once the second sections 66 have penetrated to a desired depth, cryogenic fluid (e.g., liquid nitrogen) is then delivered through the inlet channels 74 of the first sections 62, and returned through the outlet channels 78. The cryogenic fluid circulates in a continuous flow, causing rapid cooling of the distal ends 82 of the first sections 62. This cooling at the distal ends 82 is transferred to the solid, superconductive material of the second sections 66, thereby rapidly cooling the second sections 66. The second sections 66 are cooled to approximately -15° C, or to less than -15° C (e.g., between -15° C and -70° C, or between -15° C and -40° C, or between -20° C and - 40° C), so as to cause ablation of the renal veins or other tissue that is the target of the ablation. Other examples include other temperatures or ranges of temperatures.

Throughout the cooling and ablation, the catheter body 14 may remain positioned within the renal artery 46. In some examples, the catheter assembly 10 is sized and shaped such that the renal artery 46 is only partially obstructed during the procedure by the catheter assembly 10, and thus blood may continue to flow through the renal artery 46 even as ablation is occurring.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

The invention may be further described by reference to the following numbered paragraphs:
1. A catheter assembly comprising:
   an elongate catheter body defining a longitudinal axis; and
   a plurality of cooling needles configured to be extended from the catheter body to penetrate a vessel wall of a vessel in which the elongate catheter body is positioned and extend to an ablation region within tissue adjacent the vessel wall;
   wherein at least one cooling needle of the plurality of cooling needles is further configured to receive a flow of cryogenic fluid to cool the at least one cooling needle.
2. The catheter assembly of paragraph 1, wherein at least one cooling needle of the plurality of cooling needles is flexible.
3. The catheter assembly of paragraph 1 or 2, wherein at least one cooling needle of the plurality of cooling needles comprises a first second and a second section extending distally from the first section, wherein the second section defines a distal tip of the at least one cooling needle, and wherein the first section comprises a channel configured to receive the flow of cryogenic fluid.
4. The catheter assembly of paragraph 3, wherein the second section is solid and does not comprise the channel.
5. The catheter assembly of paragraph 3 or 4, wherein the second section is sized and shaped to be deployed outwardly through a distal end of the catheter body, and is configured to extend radially relative to the longitudinal axis and radially penetrate the vessel wall.
6. The catheter assembly of paragraph 5, wherein the second section is sized and shaped to extend circumferentially relative to the longitudinal axis and circumferentially penetrate the vessel wall.
7. The catheter assembly of paragraph 3 or 4, wherein the second section is sized and shaped to extend circumferentially relative to the longitudinal axis and circumferentially penetrate the vessel wall.
8. The catheter assembly of any one of paragraph 3 to 6, wherein the at least one cooling needle is pre-stressed, such that the at least one cooling needle is configured to be advanced axially through the elongate catheter body and the second section is configured to automatically move radially relative to the longitudinal axis upon deployment from the elongate catheter body.
9. The catheter assembly of any one of paragraph 3 to 7, wherein the at least one cooling needle is pre-stressed, such that the at least one cooling needle is configured to be advanced axially through the elongate catheter body and the second section is configured to automatically move circumferentially relative to the axis upon outward deployment from the catheter body.
10. The catheter assembly of any one of paragraph 3 to 9, wherein the plurality of cooling needles includes three cooling needles.
11. The catheter assembly of paragraph 10, wherein a distal end of the elongate catheter body defines three separate apertures, and wherein the second section of each of the cooling needles is sized and shaped to be deployed out of the elongate catheter body through a different one of the three separate apertures.
12. The catheter assembly of paragraph 10, wherein the distal end of the catheter body defines a single aperture, and wherein the second section of each of the cooling needles is sized and shaped to be deployed out of the catheter body through the single aperture.
13. The catheter assembly of any one of paragraph 3 to 12, wherein the second section comprises gold.
14. The catheter assembly of any one of paragraph 3 to 13, wherein the first section comprises a highly insulative material.
15. The catheter assembly of paragraph 14, wherein the first section comprises polyimide.
16. An assembled catheter comprising the components of the catheter assembly of any one of paragraph 3 to 15, wherein the plurality of cooling needles are positioned within the elongate catheter body in the assembled catheter.
17. The catheter of paragraph 16, wherein the plurality of cooling needles are stacked within the catheter body, such that a first cooling needle of the plurality of cooling needles is positioned to be deployed first, and a second cooling needle of the plurality of cooling needles is positioned to be deployed second.
18. A method of using the catheter of paragraph 16 or 17, wherein the method comprises:
   advancing the distal end of the elongate catheter body over a guidewire to a location proximate the vessel wall; and
   deploying the second sections of the plurality of cooling needles out of the distal end of the catheter body, so that the second sections move at least one of radially or circumferentially relative to the longitudinal axis and penetrate the vessel wall.
19. The method of paragraph 18, wherein the second sections penetrate at least a depth of 1 mm radially into the vasculature wall.
20. The method of paragraph 18 or 19, further comprising directing cryogenic fluid through the first sections of the cooling needles, and causing the second sections to become cooled and to ablate renal nerves attached to the vasculature wall.

Although various aspects and examples have been described in detail with reference to certain examples illustrated in the drawings, variations and modifications exist within the scope and spirit of one or more independent aspects described and illustrated.

Further disclosed herein is the subject-matter of the following clauses:
1. A catheter assembly comprising:
   an elongate catheter body defining a longitudinal axis; and
   a plurality of cooling needles configured to be extended from the catheter body to penetrate a vessel wall of a vessel in which the elongate catheter body is positioned and extend to an ablation region within tissue adjacent the vessel wall;
   wherein at least one cooling needle of the plurality of cooling needles is further configured to receive a flow of cryogenic fluid to cool the at least one cooling needle.
2. The catheter assembly of clause 1, wherein at least one cooling needle of the plurality of cooling needles is flexible.
3. The catheter assembly of clause 1 or 2, wherein at least one cooling needle of the plurality of cooling needles comprises a first section and a second section extending distally from the first section, wherein the second section defines a distal tip of the at least one cooling needle, and wherein the first section comprises a channel configured to receive the flow of cryogenic fluid.
4. The catheter assembly of clause 3, wherein the second section is solid and does not comprise the channel.
5. The catheter assembly of clause 3 or 4, wherein the second section is sized and shaped to be deployed outwardly through a distal end of the catheter body, and is configured to extend radially relative to the longitudinal axis and radially penetrate the vessel wall.
6. The catheter assembly of clause 5, wherein the second section is sized and shaped to extend circumferentially relative to the longitudinal axis and circumferentially penetrate the vessel wall.
7. The catheter assembly of clause 3 or 4, wherein the second section is sized and shaped to extend circumferentially relative to the longitudinal axis and circumferentially penetrate the vessel wall.
8. The catheter assembly of clause 3 or of any of clauses 3-6, wherein the at least one cooling needle is pre-stressed, such that the at least one cooling needle is configured to be advanced axially through the elongate catheter body and the second section is configured to automatically move radially relative to the longitudinal axis upon deployment from the elongate catheter body.
9. The catheter assembly of clause 3 or of any of clauses 3-7, wherein the at least one cooling needle is pre-stressed, such that the at least one cooling needle is configured to be advanced axially through the elongate catheter body and the second section is configured to automatically move circumferentially relative to the axis upon outward deployment from the catheter body.
10. The catheter assembly of clause 3 or of any of clauses 3-9, wherein the plurality of cooling needles includes three cooling needles.
11. The catheter assembly of clause 10, wherein a distal end of the elongate catheter body defines three separate apertures, and wherein the second section of each of the cooling needles is sized and shaped to be deployed out of the elongate catheter body through a different one of the three separate apertures.
12. The catheter assembly of clause 10, wherein a distal end of the catheter body defines a single aperture, and wherein the second section of each of the cooling needles is sized and shaped to be deployed out of the catheter body through the single aperture.
13. The catheter assembly of clause 3 or of any of clauses 3-12, wherein the second section comprises gold.
14. The catheter assembly of clause 3 or of any of clauses 3-13, wherein the first section comprises a highly insulative material.
15. The catheter assembly of clause 14, wherein the first section comprises polyimide.
16. An assembled catheter comprising the components of the catheter assembly of clause 3 or of any of clauses 3-15, wherein the plurality of cooling needles are positioned within the elongate catheter body in the assembled catheter.
17. The catheter of clause 16, wherein the plurality of cooling needles are stacked within the catheter body, such that a first cooling needle of the plurality of cooling needles is positioned to be deployed first, and a second cooling needle of the plurality of cooling needles is positioned to be deployed second.
18. A method of using the catheter of clause 16, wherein the method comprises:
   advancing a distal end of the elongate catheter body over a guidewire to a location proximate the vessel wall; and
   deploying the second sections of the plurality of cooling needles out of the distal end of the catheter body, so that the second sections move at least one of radially or circumferentially relative to the longitudinal axis and penetrate the vessel wall.
19. The method of clause 18, wherein the second sections penetrate at least a depth of 1 mm radially into a vasculature wall.
20. The method of clause 18 or of any of clauses 18-19, further comprising directing cryogenic fluid through the first sections of the cooling needles, and causing the second sections to become cooled and to ablate renal nerves attached to a vasculature wall.

## Claims

1. A catheter assembly comprising:
an elongate catheter body defining a longitudinal axis; and
a plurality of cooling needles configured to be extended from the catheter body to penetrate a vessel wall of a vessel in which the elongate catheter body is positioned and extend to an ablation region within tissue adjacent the vessel wall;
wherein at least one cooling needle of the plurality of cooling needles is further configured to receive a flow of cryogenic fluid to cool the at least one cooling needle.

2. The catheter assembly of claim 1, wherein at least one cooling needle of the plurality of cooling needles is flexible.

3. The catheter assembly of claim 1 or 2, wherein at least one cooling needle of the plurality of cooling needles comprises a first section and a second section extending distally from the first section, wherein the second section defines a distal tip of the at least one cooling needle, and wherein the first section comprises a channel configured to receive the flow of cryogenic fluid.

4. The catheter assembly of claim 3, wherein the second section is solid and does not comprise the channel.

5. The catheter assembly of claim 3 or 4, wherein the second section is sized and shaped to be deployed outwardly through a distal end of the catheter body, and is configured to extend radially relative to the longitudinal axis and radially penetrate the vessel wall.

6. The catheter assembly of claim 5, wherein the second section is sized and shaped to extend circumferentially relative to the longitudinal axis and circumferentially penetrate the vessel wall.

7. The catheter assembly of claim 3 or 4, wherein the second section is sized and shaped to extend circumferentially relative to the longitudinal axis and circumferentially penetrate the vessel wall.

8. The catheter assembly of any one of claims 3 to 6, wherein the at least one cooling needle is pre-stressed, such that the at least one cooling needle is configured to be advanced axially through the elongate catheter body and the second section is configured to automatically move radially relative to the longitudinal axis upon deployment from the elongate catheter body.

9. The catheter assembly of any one of claims 3 to 7, wherein the at least one cooling needle is pre-stressed, such that the at least one cooling needle is configured to be advanced axially through the elongate catheter body and the second section is configured to automatically move circumferentially relative to the axis upon outward deployment from the catheter body.

10. The catheter assembly of any one of claims 3 to 9, wherein the plurality of cooling needles includes three cooling needles.

11. The catheter assembly of claim 10, wherein a distal end of the elongate catheter body defines three separate apertures, and wherein the second section of each of the cooling needles is sized and shaped to be deployed out of the elongate catheter body through a different one of the three separate apertures.

12. The catheter assembly of claim 10, wherein a distal end of the catheter body defines a single aperture, and wherein the second section of each of the cooling needles is sized and shaped to be deployed out of the catheter body through the single aperture.

13. The catheter assembly of any one of claims 3 to 12, wherein the second section comprises gold.

14. The catheter assembly of any one of claim 3 to 13, wherein the first section comprises a highly insulative material.

15. The catheter assembly of claim 14, wherein the first section comprises polyimide.
